# EUROPEAN PATENT SPECIFICATION

(11) **EP 0 614 902 B1**
(45) Date of publication and mention of the grant of the patent: **11.06.1997**
(21) Application number: 94301774.9
(22) Date of filing: 11.03.1994
(51) Int. Cl.: C07F 9/50, C07F 15/00

(54) **Phosphine compounds and transition metal-phosphine complexes containing them as ligands**
Phosphinverbindungen und Übergangsmetall-Phosphin-Komplexverbindungen, die diese als Liganden enthalten
Composés phosphiniques et complexes métal de transition-phosphine les contenant comme ligands

(30) Priority: 12.03.1993 JP 52540/93
(43) Date of publication of application: 14.09.1994
(73) Proprietor: Takasago International Corporation, Tokyo 108 (JP)
(72) Inventor: Matsumura, Kazuhiko, c/o Takasago Int. Corp., Hiratsuka-shi, Kanagawa (JP); Saito, Takao, c/o Takasago Int. Corp., Hiratsuka-shi, Kanagawa (JP); Sayo, Noboru, c/o Takasago Int. Corp., Hiratsuka-shi, Kanagawa (JP); Kumobayashi, Hidemasa, c/o Takasago Int. Corp., Hiratsuka-shi, Kanagawa (JP); Takaya, Hidemasa, Kusatsu-shi, Shiga (JP)
(74) Representative: Bubb, Antony John Allen

(56) References cited:
- EP-A- 0 307 168
- EP-A- 0 408 340
- EP-A- 0 479 541
- EP-A- 0 503 884

## Description

This invention relates to novel phosphine compounds, and to transition metal complexes containing them as ligands and which are useful as catalysts for various asymmetrical synthesis reactions.

A number of transition metal complexes have hitherto been used as catalysts for organic synthesis. In particular, since noble metal complexes, though expensive, are stable and easy to handle, intensive study has been conducted on their use as a catalyst for organic synthesis. As a result, many noble metal complex catalysts have been reported to realize organic synthesis reactions that have been regarded as impossible with a conventional means.

Inter alia, many catalysts exhibiting excellent performance in asymmetric reactions are found among those composed of an optically active tertiary phosphine coordinated to a transition metal, e.g., rhodium, palladium, ruthenium or nickel. In an attempt to further improve the performance of these catalysts, plenty of phosphine compounds having a specific structure have been developed (see Nippon Kagakukai (ed.), KAGAKU SOSETSU, Vol. 32, pp. 237-238 (YUKI KINZOKU NO KAGAKU) (1982)).

U.S. Patent 5,206,399 discloses 2,2'-bis(diphenylphosphino)-5,5',6,6',7,7',8,8'-octahydro-1,1'-binaphthyl and asymmetric hydrogenation using a transition metal complex containing the phosphine as a ligand.

A great number of special phosphine compounds have thus been developed for the purpose of providing a catalyst having higher performance in asymmetric syntheses. However, they are not always satisfactory in selectivity, conversion, catalytic activity, asymmetric yield, and the like depending on the substrate or the reaction to which they are applied.

An object of the present invention is to provide a novel phosphine compound providing a complex having higher catalytic performance than the conventional catalysts.

As a result of extensive investigations, the present inventors have found that a transition metal complex containing a novel phosphine compound having a 5,5',6,6',7,7',8,8'-octahydro-1,1'-binaphthyl skeleton (i.e., bitetrahydronaphthalene group) and a phosphine structure and a phosphite structure per molecule is considerably superior to the known optically active phosphine-transition metal complexes in asymmetric yield and selectivity in asymmetric syntheses. The present invention has been completed based on this finding.

The present invention relates to a phosphine compound represented by formula (I): 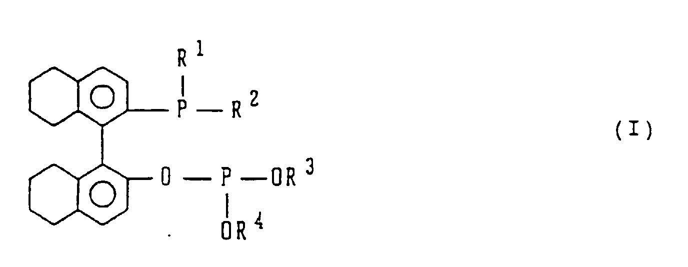 wherein R¹ and R², which may be the same or different, each represent a phenyl group or a phenyl group substituted with a halogen atom or a lower alkyl group or they are taken together to form a divalent hydrocarbon group; and R³ and R⁴, which may be the same or different, each represent a lower alkyl group, a phenyl group or a phenyl group substituted with a halogen atom, a lower alkyl group or a lower alkoxy group or they are taken together to form a divalent hydrocarbon group.

The present invention also relates to a transition metal-phosphine complex containing the phosphine compound of formula (I) as a ligand.

In formula (I), the lower alkyl group includes a methyl group, an ethyl group, a propyl group, an isopropyl group, a butyl group, an isobutyl group, a sec-butyl group, a t-butyl group, a pentyl group, and a hexyl group. The halogen atom includes a chlorine atom, a bromine atom, an iodine atom, and a fluorine atom. The lower alkoxy group includes a methoxy group, an ethoxy group, a propoxy group, an isopropoxy group, a butoxy group, an isobutoxy group, a sec-butoxy group, and a t-butoxy group. The divalent hydrocarbon group includes biarylene groups, e.g., a substituted or unsubstituted biphenyl group, a substituted or unsubstituted binaphthyl group, a substituted or unsubstituted biphenanthryl group, and a substituted or unsubstituted bianthryl group; and straight chain or branched and saturated or unsaturated alkylene groups having 2-6 carbon atoms, e.g., an ethylene group, a trimethylene group, a tetramethylene group, a pentamethylene group, a 1,4-dimethyltetramethylene group, a 1,3-butadienylene group, and a 1,4-dimethyl-1,3-butadienylene group. Substituents which may be on the above biarylene groups include a lower alkyl group, a halogen atom, and a lower alkoxy group. The transition metal in the transition metal-phosphine complex includes rhodium, ruthenium, palladium, iridium, platinum, cobalt, and nickel. In the present invention, rhodium, ruthenium, iridium and palladium are preferred, and rhodium, iridium and palladium are particularly preferred as the transition metal.

The phosphine compound represented by formula (I) can be prepared through, for example, the following reaction scheme: 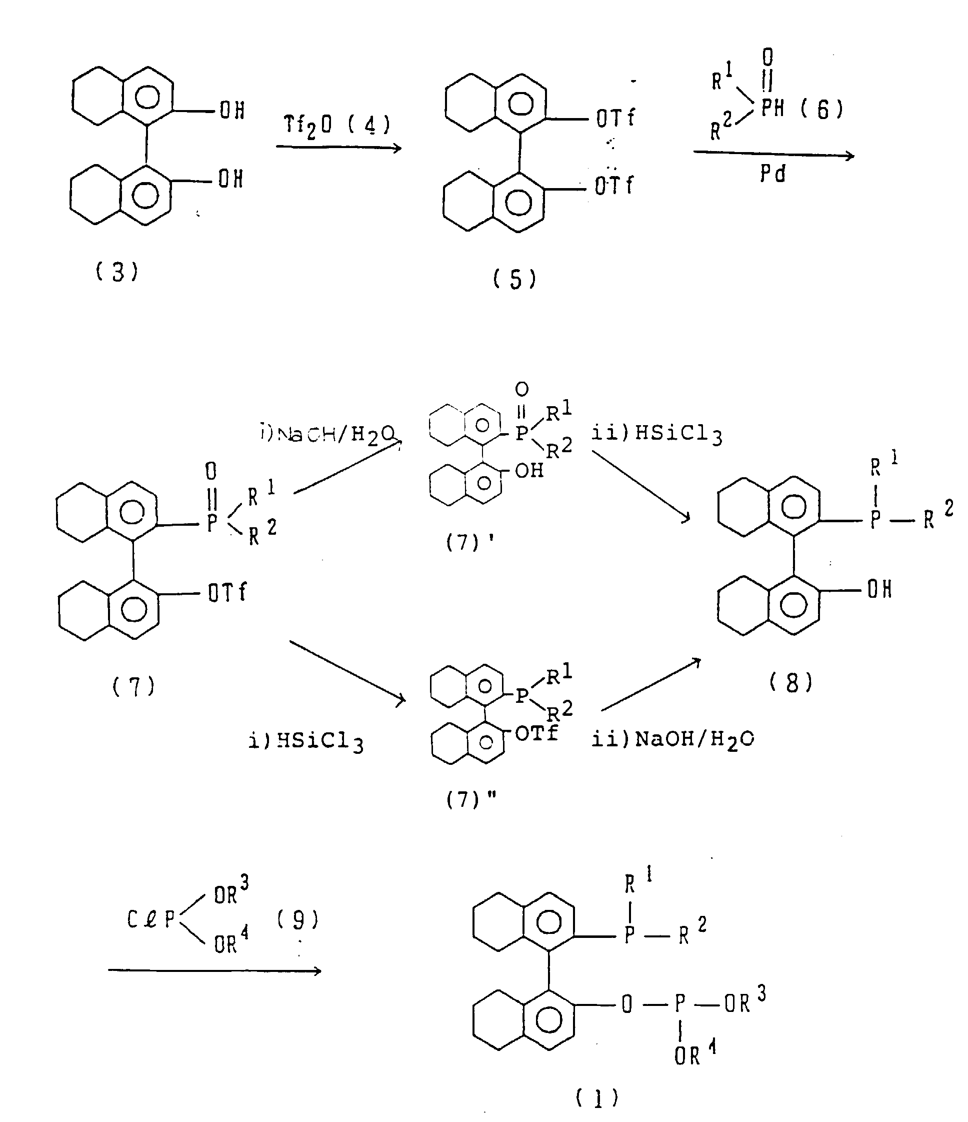 wherein R¹, R², R³, and R⁴ are as defined above; and Tf represents a trifluoromethanesulfonyl group.

In detail, 2,2'-dihydroxy-5,5',6,6',7,7',8,8'-octahydro-1,1'-binaphthyl (3), which is obtained by hydrogenation of 1,1'-bi-2-naphthol in the presence of a platinum oxide catalyst, is reacted with trifluoromethanesulfonic acid anhydride (4) in accordance with the process described in Tetrahedron Letters, Vol. 31, pp. 6321-6324 (1990) to obtain 2,2'-bis(trifluoromethanesulfonyloxy)-5,5',6,6',7,7',8,8'-octahydro-1,1'-binaphthyl (5). Compound (5) is reacted with phosphine oxide (6) in the presence of a palladium catalyst to obtain compound (7). Compound (7) is first reduced by trichlorosilane in the presence of triethylamine and then hydrolyzed or first hydrolyzed and then reduced to obtain 2-diphenylphosphino-2'-hydroxy-5,5',6,6',7,7',8,8'-octahydro-1,1'-binaphthyl (8). Compound (8) is reacted with a chlorophosphite derivative (9) in the presence of triethylamine to obtain an optically active phosphine compound of formula (I).

The optically active 1,1'-bi-2-naphthol which can be used as a starting material can easily be prepared in high yield and with high optical purity by reacting optically active O,O'-dimethyl-N,N'-tetramethyltartaric acid amide prepared from natural or non-natural tartaric acid with an optical isomer of racemic binaphthol to form a clathrate complex and decomposing the complex.

The transition metal-phosphine complex according to the present invention can be prepared according to known processes, for example, the process described in Nippon Kagakukai (ed.), JIKKEN KAGAKU KOZA, 4th Ed., Vol. 18, pp. 254-438.

The transition metal complexes according to the present invention can be synthesized by reacting the phosphine compound (I) with a transition metal compound in an appropriate solvent, such as toluene, benzene, hexane, heptane, isooctane, decane, tetrahydrofuran, diethylether, acetone, methyl ethyl ketone, methylene chloride, and chloroform, and more preferably toluene, benzene, tetrahydrofuran, and methylene chloride.

Specific examples of the transition metal-phosphine complex of the present invention are shown below. In the following formulae, COD represents 1,5-cyclooctadiene; acac represents acetylacetonato; and L represents the phosphine compound of formula (I).
Ni(L)₂, PdCℓ₂(L), (π-allyl)PdCℓ(L), [(π-allyl)Pd(L)]CℓO₄,[(π-allyl)Pd(L)]PF₆, [(π-allyl)Pd(L)]BF₄, Pt(L)₂, Rh(COD)Cℓ(L), [Rh(COD)(L)]CℓO₄, [Rh(COD)(L)]PF₆, [Rh(COD)(L)]BF₄, Rh(acac)(L), Ir(COD)Cℓ(L), [Ir(COD)(L)]CℓO₄, [Ir(COD)(L)]PF₆, [Ir(COD)(L)]BF₄, [Ru(C₆H₆)Cℓ(L)]Cℓ, [Ru(p-cymene)Cℓ(L)]Cℓ, and (methallyl)Ru(L).

The octahydro binaphthyl skeleton of the phosphine compound (I) may be an optically active compound or a racemate, both of which are included in the scope of the present invention.

When the transition metal-phosphine complex of the present invention is used as a catalyst for, for example, asymmetric hydroformylation of an olefin, a desired product can be obtained in a high asymmetric yield that has never been reached by conventional techniques. By choosing either one of the phosphine isomers, having a plus (+) or (-) optical rotation, and using a transition metal-phosphine complex containing the selected phosphine isomer, a compound having a desired absolute configuration can be obtained by asymmetric synthesis in a high asymmetric yield.

Where rhodium is used as a transition metal, the same reaction results as obtained in using the above-enumerated rhodium-optically active phosphine complex can also be obtained by using the rhodium-phosphine complex in combination with the optically active phosphine compound in excess or a combination of a commercially available rhodium complex, e.g., [Rh(CO)₂(acac)], as a catalyst precursor and 2 to 4 equivalents, to rhodium, of the optically active phosphine compound of formula (I).

Further, the same reaction results may also be obtained by using a rhodium-carbonyl cluster, e.g., Rh₆(CO)₁₆ or Rh₄(CO)₁₂, as a catalyst precursor. A rhodium-carbonyl cluster Rhₙ(CO)ₙ may previously be reacted with a halogen (e.g., chlorine, bromine or iodine) and the reaction product may be used in combination with the optically active phosphine compound of formula (I).

The transition metal-phosphine complex of the present invention is usually a mononuclear complex having a single transition metal atom and, in some cases, may be a polynuclear complex having two or more transition metal atoms.

The present invention will now be illustrated in greater detail with reference to Examples and Reference Examples, but the present invention should not be construed as being limited thereto. All the percents are given by weight unless otherwise specified.

Measuring instruments used in Examples are as follows.
Nuclear Magnetic Resonance Spectrum (NMR):
Model AM-400 manufactured by Bruker Inc.
Internal Standard:
¹H-NMR: tetramethylsilane
External Standard:
³¹P-NMR: 85% phosphoric acid
Optical Purity:
GC-15A Gas Chromatograph manufactured by Shimadzu Corporation
Column: Chiral capillary column, CHROMPACK β-236M
Chemical Purity:
Hitachi 263-30 Gas Chromatograph manufactured by Hitachi, Ltd.
Optical Rotation:
Model DIP-360 manufactured by JASCO Inc.

### EXAMPLE 1

### Synthesis of (R)-2-Diphenylphosphino-5,5',6,6',7,7',8,8'-octahydro-1,1'-binaphthalen-2'-yloxy-((S)-1,1'-binaphthalene-2,2'-diyldioxy)phosphine

### (1) Synthesis of (R)-5,5',6,6',7,7',8,8'-Octahydro-1,1'-bi-2-naphthol:

In a 200 mℓ autoclave were charged 3.00 g (10.5 mmol) of (R)-2,2'-dihydroxy-1,1'-binaphthyl, 360 mg of platinum dioxide, and 75 mℓ of acetic acid, and the mixture was stirred at 5°C for 18 hours under a hydrogen pressure of 3 atm and then at 18°C for 7 days under a hydrogen pressure of 10 atm. Ethyl acetate was added to the reaction mixture, and the mixture was filtered through Celite. Water was added to the filtrate, followed by liquid-liquid separation. The separated organic layer was washed twice with a saturated sodium hydrogencarbonate aqueous solution, followed by liquid-liquid separation. The organic layer was dried over magnesium sulfate, and the solvent was removed by distillation under reduced pressure to obtain 3.03 g (percent yield: 98%) of the titled binaphthol. Optical purity: ≥99 %ee.

### (2) Synthesis of (R)-2,2'-Bis(trifluoromethanesulfonyloxy)-5,5',6,6',7,7'8,8'-octahydro-1,1'-binaphthyl:

In a 50 mℓ four-necked flask was put a solution of 7.36 g (25 mmol) of (R)-5,5',6,6',7,7',8,8'-octahydro-1,1'-bi-2-naphthol and 6 mℓ (74 mmol) of pyridine in 50 mℓ of methylene chloride, and 15.5 g (55 mmol) of trifluoromethanesulfonic acid anhydride was added to the solution at 0°C, followed by stirring at room temperature for 23 hours to complete the reaction. After completion of the reaction, the solvent was removed by distillation, and the residue was purified by silica gel column chromatography using a 10:1 (by volume; hereinafter the same) mixture of hexane and ethyl acetate as a developing solvent to obtain 12.88 g (percent yield: 92%) of (R)-2,2'-bis(trifluoromethanesulfonyloxy)-5,5',6,6',7,7',8,8'-octahydro-1,1'-binaphthyl.

### (3) Synthesis of (R)-2-Diphenylphosphinyl-2'-trifluoromethanesulfonyloxy-5,5',6,6',7,7',8,8'-octahydro-1',1-binaphthyl:

In a 50 mℓ flask was charged a solution of 2.68 g (4.80 mmol) of (R)-2,2'-bis(trifluoromethanesulfonyloxy)-5,5',6,6',7,7',8,8'-octahydro-1,1'-binaphthyl obtained in (2) above and 1.99 g (9.04 mmol) of diphenylphosphine oxide in 20 mℓ of dimethyl sulfoxide in an argon stream. To the solution were added 108 mg (0.480 mmol) of palladium acetate, 205 mg (0.480 mmol) of 1,4-bis(diphenylphosphino)butane, 5.1 mℓ of ethyldiisopropylamine, and 33 mg (0.491 mmol) of sodium formate, and the mixture was stirred at room temperature for 20 minutes and then at 90°C for 19 hours. After cooling to room temperature, 250 mℓ of ethyl ether and 150 mℓ of water were added to the reaction mixture, followed by stirring and liquid-liquid separation.

The organic layer thus separated was washed successively with four 125 mℓ portions of water, two 125 mℓ portions of 5% dilute hydrochloric acid, two 50 mℓ portions of water, a 125 mℓ portion of a saturated sodium hydrogencarbonate aqueous solution, and a 125 mℓ portion of a saturated sodium chloride aqueous solution and dried over anhydrous magnesium sulfate. The solvent was distilled off, and the residue was purified by silica gel column chromatography using a 2:1 to 1:1 mixture of hexane and ethyl acetate as a developing solvent to obtain 2.76 g (percent yield: 94%) of the titled compound.

### (4) Synthesis of (R)-2-Diphenylphosphino-2'-hydroxy-5,5',6,6',7,7',8,8'-octahydro-1,1'-binaphthyl:

In a mixed solvent of 3 mℓ of 1,4-dioxane and 2 mℓ of methanol was dissolved 405.4 mg (0.664 mmol) of (R)-2-diphenylphosphinyl-2'-trifluoromethanesulfonyloxy-5,5',6,6',7,7',8,8'-octahydro-1,1'-binaphthyl, and 3 mℓ of a 3N sodium hydroxide aqueous solution was added thereto, followed by stirring at room temperature for 15 hours. The reaction mixture was made acidic with concentrated hydrochloric acid and poured into 50 mℓ of water. The thus precipitated solid was collected by filtration to obtain a crude product. The crude product was dissolved in 22 mℓ of xylene in a 50 mℓ four-necked flask, and 1.21 g (12 mmol) of triethylamine and 1.62 g (12 mmol) of trichlorosilane were added to the solution, followed by stirring at 120°C for 17 hours. After cooling to room temperature, 4.4 mℓ of a 35% sodium hydroxide aqueous solution was carefully added to the reaction mixture. The mixture was stirred for 2 hours, followed by liquid-liquid separation.

The separated organic layer was washed with two 30 mℓ portions of a saturated sodium chloride aqueous solution, dried over anhydrous magnesium sulfate, and distilled to remove the solvent. The residue was purified by silica gel column chromatography using a 5:1 mixture of hexane and ethyl acetate as a developing solvent to obtain 245.8 mg (percent yield: 80%) of the titled compound.

### (5) Synthesis of (S)-1,1'-Binaphthalene-2,2'-diyldioxychlorophosphine:

In a 50 mℓ flask equipped with a reflux condenser were charged 4.94 g (17.3 mmol) of (S)-1,1'-bi-2-naphthol and 113 g (830 mmol) of phosphorus trichloride in an argon stream, and the mixture was heated at reflux for 4 hours, followed by cooling to room temperature. The unreacted phosphorus trichloride was removed by distillation under reduced pressure to give 5.56 g (percent yield: 92%) of the titled compound as a white crystal.

### (6) Synthesis of (R)-2-Diphenylphosphino-5,5',6,6',7,7',8,8'-octahydro-1,1'-binaphthalene-2'-yloxy-((S)-1,1'-binaphthalene-2,2'-diyldioxy)phosphine:

In a 100 mℓ flask, 0.438 g (0.946 mmol) of (R)-2-diphenylphosphino-2'-hydroxy-5,5',6,6',7,7',8,8'-octahydro-1,1'-binaphthyl and 662 mg (1.89 mmol) of (S)-1,1'-binaphthalene-2,2'-diyldioxychlorophosphine were dissolved in 30 mℓ of ethyl ether, and 191 mg (1.89 mmol) of triethylamine was added to the solution at 0°C. The mixture was stirred at room temperature for 15 hours, and 20 mℓ of water was added thereto to cease the reaction.

After liquid-liquid separation, the organic layer separated was dried over anhydrous magnesium sulfate, and the solvent was removed by distillation to obtain a crude product. Purification by silica gel column chromatography using a 1:1 mixture of hexane and benzene as a developing solvent yielded 719.4 mg (percent yield: 98%) of (R)-2-diphenylphosphino-5,5',6,6',7,7',8,8'-octahydro-1,1'-binaphthalen-2'-yloxy-((S)-1,1'-binaphthalene-2,2'-diyldioxy)phosphine (hereinafter referred to as (R,S)-OcH-BINAPHOS) as a white crystal.
³¹P-NMR (CDCℓ₃) δ:
-14.8 (d, J=42.7Hz), 146.8 (d, J=42.7Hz)

### EXAMPLE 2

### Synthesis of (R)-2-Diphenylphosphino-5,5',6,6',7,7',8,8'-octahydro-1,1'-binaphthalen-2'-yloxy-((S)-5,5',6,6',7,7'-8,8'-octahydro-1,1,'-binaphthalene-2,2'-diyldioxy)phosphine

### (1) Synthesis of (S)-5,5',6,6',7,7',8,8'-Octahydro-1,1'-binaphthalene-2,2'-diyldioxychlorophosphine:

In a 50 mℓ flask were charged 5.12 g (17.3 mmol) of (S)-5,5',6,6',7,7',8,8'-octahydro-1,1'-bi-2-naphthol and 113 g (830 mmol) of phosphorus trichloride in an argon stream, and the mixture was heated at reflux for 4 hours, followed by cooling to room temperature. The unreacted phosphorus trichloride was removed by distillation under reduced pressure to give 5.7 g (percent yield: 92%) of the titled compound as a white crystal.

### (2) Synthesis of (R)-2-Diphenylphosphino-5,5',6,6',7,7',8,8'-octahydro-1,1'-binaphthalen-2'-yloxy-((S)-5,5',6,6',7,7',8,8'-octahydro-1,1'-binaphthalene-2,2'-diyldioxy)phosphine (hereinafter referred to as (R,S)-(OcH)₂-BINAPHOS):

(R,S)-(OcH)₂-BINAPHOS) was obtained in a percent yield of 91% in the same manner as in Example 1-(6), except for replacing (S)-1,1'-binaphthalene-2,2'-diyldioxychlorophosphine with (S)-5,5',6,6',7,7',8,8'-octahydro-1,1'-binaphthalene-2,2'-diyldioxychlorophosphine.
³¹P-NMR (CDCℓ₃) δ:
-14.6 (d, J=39.7Hz), 138.2 (d, J=39.7Hz)

### EXAMPLE 3

### Synthesis of Rh((R,S)-OcH-BINAPHOS)(acac)

In a 20 mℓ Schlenk's tube were charged 19.4 mg (0.025 mmol) of (R,S)-OcH-BINAPHOS and 6.5 mg (0.025 mmol) of Rh(CO)₂(acac) and dissolved with 5 mℓ of methylene chloride. After stirring at room temperature for 5 minutes, the solvent was removed by distillation under reduced pressure to obtain Rh((R,S)-OcH-BINAPHOS)(acac) as a yellow solid in a percent yield of 100%.
³¹P-NMR (CDCℓ₃) δ:
46.2 (dd, Jₚ₋ₚ=82.4Hz, J_{Rh-p}=174.0Hz), 160.7 (dd, J_{Rh-p}=331.0Hz)

### EXAMPLE 4

### Synthesis of Rh((R,S)-(OcH)₂-BINAPHOS(acac)

The titled compound was obtained in a percent yield of 100% in the same manner as in Example 3, except for replacing (R,S)-OcH-BINAPHOS with (R,S)-(OcH)₂-BINAPHOS.
³¹P-NMR (CDCℓ₃) δ:
48.3 (dd, Jₚ₋ₚ=83.9Hz, J_{Rh-p}=174.0Hz), 161.8 (dd, J_{Rh-p}=331.1HZ)

### EXAMPLE 5

Complexes shown in Table 1 below were prepared in the same manner as in the foregoing Examples.

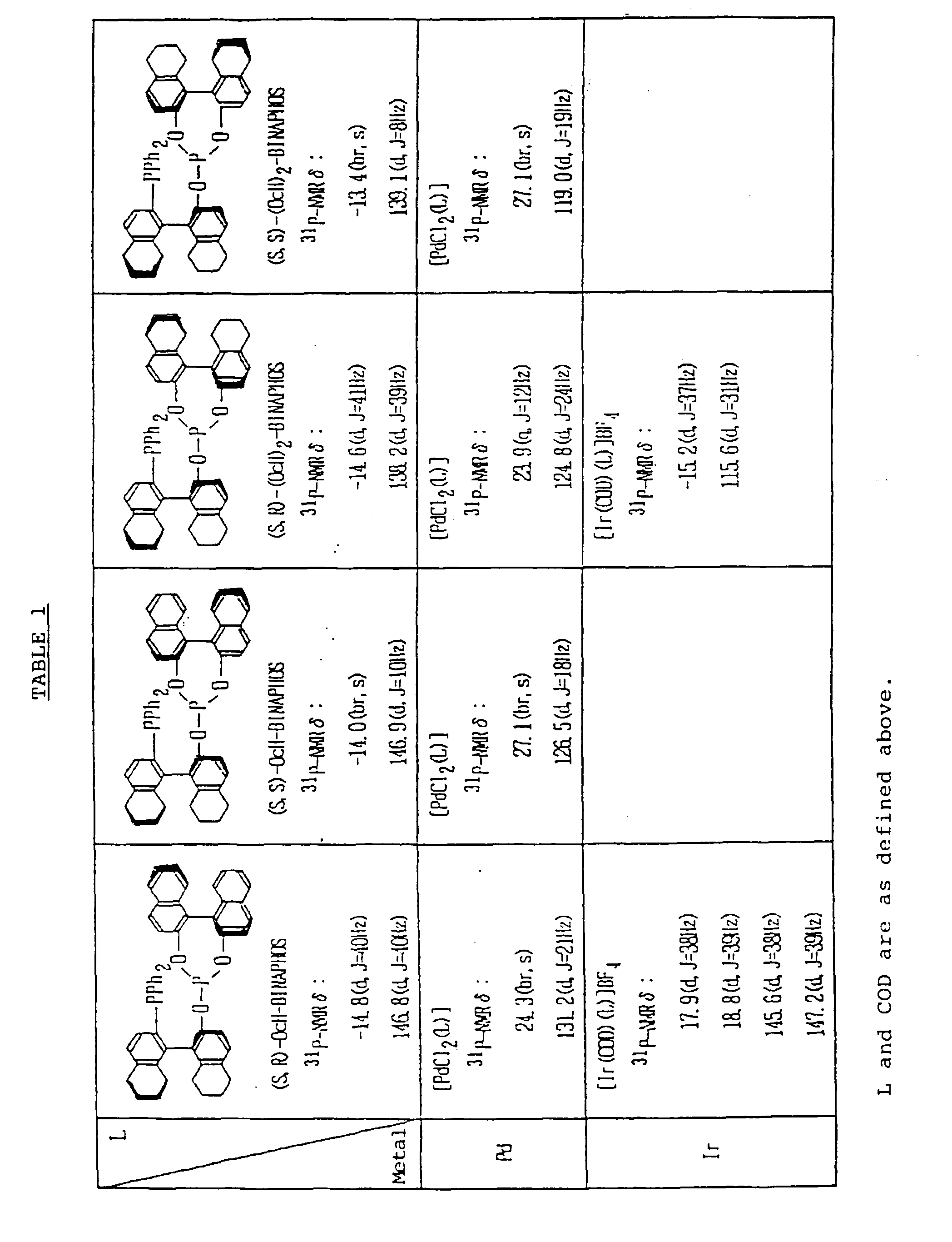

### REFERENCE EXAMPLE 1

### Asymmetric Hydroformylation of Vinyl Acetate

In a 50 mℓ autoclave were put 761 mg (8.84 mmol) of vinyl acetate, 8.6 mg (0.00884 mmol) of Rh((R,S)-OcH-BINAPHOS)(acac), and 8.3 mℓ of benzene. Hydrogen and carbon monoxide were introduced into the autoclave to a total pressure of 100 atm with the hydrogen pressure being 50 atm, and the mixture was stirred at 60°C for 44 hours to obtain a mixture of 2-acetoxypropanal and 3-acetoxypropanal at a ratio of 88.6:11.4 as determined by ¹H-NMR. The conversion of vinyl acetate was 72% as determined by ¹H-NMR. The resulting aldehyde was converted to the corresponding carboxylic acid by Jones oxidation and analyzed by optically active gas chromatography (CHROMPACK, CP-Cyclodextrin β-236-m-19; measured at 145°C). As a result, the optical purity of the 2-acetoxypropanal was found to be 90 %ee.

### REFERENCE EXAMPLE 2

### Hydroformylation of Styrene

In a 50 mℓ autoclave were charged 1.04 g (10 mmol) of styrene, 9.8 mg (0.01 mmol) of Rh((R,S)-OcH-BINAPHOS)(acac), and 10 mℓ of benzene. Hydrogen and carbon monoxide were introduced into the autoclave to a total pressure of 100 atm with the hydrogen pressure being 50 atm, and the mixture was stirred at 60°C for 100 hours. After completion of the reaction, the temperature was lowered to room temperature, and the pressure was relieved. The solvent was removed by distillation to obtain a mixture of 2-phenylpropanal and 3-phenylpropanal. The mixing ratio and the optical purity of the product were measured in the same manner as in Reference Example 1. As a result, the conversion of styrene was 100%, the 2-phenylpropanal to 3-phenylpropanal ratio was 92.4:7.6, and the optical purity of the 2-phenylpropanal was 92.7 %ee.

### REFERENCE EXAMPLE 3

### Hydroformylation of Styrene

Styrene was hydroformylated in the same manner as in Reference Example 2, except for using Rh((R,S)-(OcH)₂- BINAPHOS)(acac) as a catalyst. As a result, the conversion of styrene was 84%, and the product was a mixture of 91.7% of 2-phenylpropanal and 8.3% of 3-phenylpropanal. The optical purity of the 2-phenylpropanal was 88.9 %ee.

As described above, the transition metal-phosphine complex according to the present invention can be used as a catalyst for asymmetric synthesis to produce a purposed compound having a desired absolute configuration at a high optical purity in a high yield.

## Claims

1. A phosphine compound represented by formula (I): 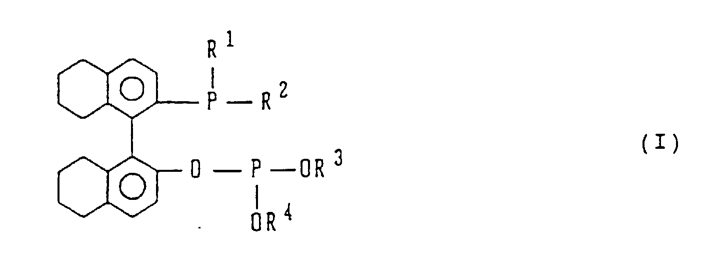 wherein R¹ and R², which may be the same or different, each represent a phenyl group or a phenyl group substituted with a halogen atom or a lower alkyl group or they are taken together to form a divalent hydrocarbon group; and R³ and R⁴, which may be the same or different, each represent a lower alkyl group, a phenyl group or a phenyl group substituted with a halogen atom, a lower alkyl group or a lower alkoxy group or they are taken together to form a divalent hydrocarbon group.

2. A transition metal-phosphine complex containing a phosphine compound represented by formula (I) as set forth and defined in Claim 1, as a ligand.

3. A transition metal-phosphine complex as claimed in Claim 2, wherein said transition metal is selected from rhodium, ruthenium, palladium, iridium, platinum, cobalt and nickel.

## Patentansprüche

1. Phosphinverbindung der Formel (I): 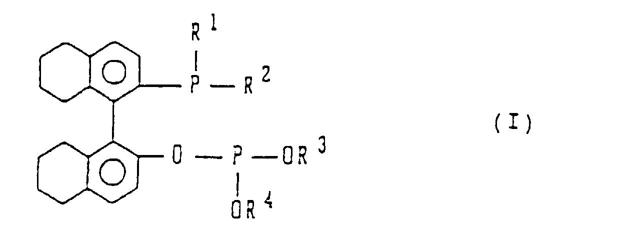 in der R₁ und R₂, die gleich oder verschieden sein können, jeweils eine Phenylgruppe oder eine Phenylgruppe, die mit einem Halogenatom oder einem Niederalkylrest substituiert ist, bedeuten oder zusammengenommen, einen zweiwertigen Kohlenwasserstoffrest bilden, und R³ und R⁴, die gleich oder verschieden sein können, jeweils einen Niederalkylrest, eine Phenylgruppe oder eine Phenylgruppe, die mit einem Halogenatom, einem Niederalkylrest oder einem Niederalkoxyrest substituiert ist, bedeuten oder zusammengenommen, einen zweiwertigen Kohlenwasserstoffrest bilden.

2. Übergangsmetallphosphinkomplex, enthaltend eine Phosphinverbindung der Formel (I), wie in Anspruch 1 beschrieben und definiert, als einen Liganden.

3. Übergangsmetallphosphinkomplex nach Anspruch 2, wobei das Übergangsmetall aus Rhodium, Ruthenium, Palladium, Iridium, Platin, Kobalt und Nickel ausgewählt ist.

## Revendications

1. Composé de phosphine représenté par la formule (I): 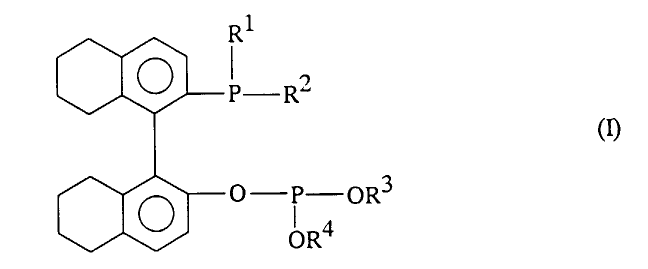 où R¹ et R² qui peuvent être identiques ou différents, représentent chacun un groupe phényle ou un groupe phényle substitué par un atome d'halogène ou un groupe alkyle inférieur ou ils peuvent former ensemble un groupe hydrocarbure divalent ; et R³ et R⁴ qui peuvent être identiques ou différents, représentent chacun un groupe alkyle inférieur, un groupe phényle ou un groupe phényle substitué par un atome d'halogène, un groupe alkyle inférieur ou un groupe alcoxy inférieur ou ils peuvent former ensemble un groupe hydrocarbure divalent.

2. Complexe phosphine-métal de transition contenant un composé phosphine représenté par la formule (I) comme c'est indiqué ci-dessus et défini par la revendication 1, comme ligand.

3. Complexe phosphine-métal de transition selon la revendication 2, où ledit métal de transition est choisi parmi les rhodium, ruthénium, palladium, iridium, platine, cobalt et nickel.
